# EUROPEAN PATENT APPLICATION

(11) **EP 2 692 852 A1**
(43) Date of publication of application: **05.02.2014**
(21) Application number: 12763447.5
(22) Date of filing: 02.04.2012
(51) Int. Cl.: C12M 3/00, A61L 27/00

(54) **CELL SHEET TRANSPLANTATION JIG AND METHOD FOR UTILIZING SAME**

(30) Priority: 31.03.2011 JP 2011092461
(71) Applicant: CellSeed Inc., Tokyo 162-0056 (JP); Osaka University, Suita-shi, Osaka 565-0871 (JP)
(72) Inventor: MIYAGAWA, Shigeru, Suita-shi Osaka 565-0871 (JP); SAITO, Atsuhiro, Suita-shi Osaka 565-0871 (JP); SAWA, Yoshiki, Suita-shi Osaka 565-0871 (JP); MIZUTANI, Manabu, Tokyo 162-0056 (JP); HARADA, Akima, Tokyo 162-0056 (JP)
(74) Representative: HOFFMANN EITLE
(86) International application number: PCT/JP2012/059003
(87) International publication number: WO 2012/133900

(57) **Abstract**

A cell sheet transplantation device having a plane for transplanting a sheet of cultured cells, the device comprising, in the plane in the same direction, (1) a planar surface portion for capturing a cell sheet while maintaining a sheet-shaped form, and (2) suction holes for immobilizing a transplantation site by suction, the suction holes being positioned around the planar surface portion. By using the cell sheet transplantation device, a cultured cell sheet can be detached effectively, and the detached cultured cell sheet can be transplanted in an effective and simple manner.

## Description

### TECHNICAL FIELD

The present invention relates to transplantation devices for cultured cell sheets in the fields of biology, medical science, and others, as well as methods for using the same.

### BACKGROUND ART

Today, animal cell culture technologies have made remarkable progress, and research and development targeted for animal cells have also been being widely made in various fields. The ways of using targeted animal cells have been expanded. At the beginning of development, only commercialization of cells themselves and their products was conducted, but now through analysis of cells themselves and proteins present on their surface layer, they are being put to various applications, including design of efficacious pharmaceutical products, and treatment of diseased sites by regenerating a patient's cells *in vitro* or enhancing their functions before returning them to the patient's body. At present, technologies for culturing, evaluating, analyzing, and using animal cells are one of the technical fields that attract the attention of researchers. In this connection, many types of animal cells including human cells are anchorage-dependent. That is, in order to culture animal cells *in vitro,* they must be temporarily attached to the surface of a substrate. Also, there is arising a need that cultured cells must be detached from the substrate while not disintegrating but maintaining the form which they took during culture on the surface of the substrate.

Particularly speaking of the technology for regenerating a patient's cells *in vitro,* organ transplantation, which aims to replace a difficult-to-treat organ with another person's organ, has been generalized in recent years. The organs to which this technique is applied have been greatly diversified as exemplified by skin, cornea, kidney, liver, and heart, and the prognosis of this technique has been significantly improved, so the regenerative medicine has been being established as one of medical technologies. Taking a corneal transplantation as an example: in Japan, the eye bank was organized and corneal transplantation activities started about 50 years ago. However, the number of donors is still small, and it is said that there are about twenty thousand patients per year in Japan alone who need a corneal transplantation but only about a tenth of them, i.e., about two thousand patients, actually undergo this therapy. The current state is that in spite of the presence of the almost established corneal transplantation technology, there is a need for a next medical technology due to the problem of lack of donors. Against this backdrop, there was developed a technology to try to culture a patient's normal cells to a desired size and transplant the cultured cells.

As another example, JP H05-192138 discloses a dermal cell culture method in which dermal cells are placed on a cell culture support having a substrate surface coated with a polymer whose upper or lower critical solution temperature in water is in the range of 0 to 80°C, the dermal cells are cultured at a temperature either below the upper critical solution temperature or above the lower critical solution temperature, and then the temperature is brought to a temperature either above the upper critical solution temperature or below the lower critical solution temperature, so that the cultured dermal cells are detached. In this method, by changing the temperature, cells are detached from the culture substrate coated with the temperature-responsive polymer, but this method is poor in detachability and often produces cell sheets with structural defects. Thus, it is difficult to apply the method disclosed in JP H05-192138 to *in vitro* construction of a myocardium-like tissue.

Further, WO 02/08387 reports the following findings: myocardial tissue cells are cultured on a cell culture support having a substrate surface coated with a temperature-responsive polymer to produce a myocardium-like cell sheet, the temperature of a culture solution is brought to a temperature either above the upper critical solution temperature or below the lower critical solution temperature, the cultured, stratified cell sheet is brought into close contact with a polymer membrane, and the cell sheet is detached together with the polymer membrane, whereby a cell sheet can be constructed which has few structural defects and which is furnished with several capabilities of an *in vitro* myocardium-like tissue; and the resulting sheet is structured three-dimensionally using a specified method to thereby construct a three dimensional structure composed of such sheets. However, even in this method, stratifying of myocardium-like cell sheets cannot be conducted by simple operations. Thus, there have been strong needs for a technology that enables simpler and more accurate stratifying of cell sheets, as well as for a method of transplanting a cell sheet stack onto a curved transplantation surface, specifically a cardiac tissue.

In order to solve the above-mentioned problems, JP 2005-176812 discloses a technology relating to a cell sheet transplantation jig having a cell adhesion surface. By using this jig, cultured cells can be detached from a cell culture substrate and then the detached cultured cells can be attached again. However, the jig disclosed therein is intended for laminating cell sheets and does not adequately function as the above-mentioned method for transplanting a cell sheet laminate onto a curved transplantation surface.

### CITATION LIST

### PATENT DOCUMENTS

Patent Document 1: Japanese Unexamined Patent Application Publication No. H05-192138
Patent Document 2: International Patent Application Publication No. WO 02/08387
Patent Document 3: Japanese Unexamined Patent Application Publication No. 2005-176812

### SUMMARY OF INVENTION

### TECHNICAL PROBLEM

The present invention has been made with the intension to solve the above-mentioned problems with the prior art. More specifically, this invention is intended to provide a novel cell sheet transplantation device based on an idea completely different from the prior art. This invention is also intended to provide a method for using said transplantation device.

### SOLUTION TO PROBLEM

In order to solve the above-mentioned problems, the present inventors have made research and development with investigations being conducted from various aspects. As a result, the inventors have found that an area around a transplantation site is immobilized by suction through suction holes of a cell sheet transplantation device, so that a cell sheet present on a cell sheet capture surface of the cell sheet transplantation device can be brought into uniform contact with the transplantation site. The inventors have also found that a force of adhesion between a cell adhesion surface of the cell sheet transplantation device and the cultured cells is weakened to thereby reattach the detached cultured cells onto the transplantation site. The present invention has been completed on the basis of these findings.

More specifically, the present application provides the following inventions:
(a) A cell sheet transplantation device having a plane for transplanting a sheet of cultured cells, the device comprising, in the plane in the same direction, (1) a planar surface portion for capturing a cell sheet while maintaining a sheet-shaped form, and (2) suction holes for immobilizing a transplantation site by suction, the suction holes being positioned around the planar surface portion.
(b) The cell sheet transplantation device as recited in (a), wherein the cell sheet transplantation device is plate-shaped.
(c) The cell sheet transplantation device as recited in (a) or (b), wherein the cell sheet transplantation device is an elastic body.
(d) The cell sheet transplantation device as recited in any one of (a) to (c), wherein the planar surface portion for capturing a cell sheet is a cell adhesion surface for detaching cells cultured on a surface of a cell culture substrate and then reattaching the detached cultured cells onto another site.
(e) The cell sheet transplantation device as recited in any one of (a) to (d), wherein the planar surface portion for capturing a cell sheet has suction holes for immobilizing a cell sheet by suction.
(f) The cell sheet transplantation device as recited in any one of (a) to (e), wherein the planar surface portion for capturing a cell sheet is a convex-shaped elastic body which is brought into contact with the surface of the cell culture substrate in such a way that it starts contacting at a tip of a convex portion of the cell adhesion surface until it finally establishes contact with the whole of a given surface of the cell culture substrate.
(g) The cell sheet transplantation device as recited in any one of (a) to (f), wherein the planar surface portion for capturing a cell sheet has attached thereto one or two or more of a cell adhesive protein, a cell adhesive peptide, and a hydrophilic polymer.
(h) The cell sheet transplantation device as recited in (g), wherein the cell adhesive protein is composed of one or two or more of fibrin gel, fibronectin, laminin, collagen, and gelatin.
(i) The cell sheet transplantation device as recited in (g), wherein the hydrophilic polymer is a hydrous gel.
(j) The cell sheet transplantation device as recited in (g), wherein the hydrophilic polymer is a temperature-responsive polymer.
(k) A method for using a cell sheet transplantation device, the method comprising: (1) attaching cultured cells present on a cell culture substrate to a planar surface portion for capturing a cell sheet, which is provided on the cell sheet transplantation device as recited in any one of (a) to (j), to thereby detach a sheet of the cultured cells from the cell culture substrate; (2) transferring the cell sheet transplantation device to a transplantation site; (3) immobilizing the transplantation site by suction through suction holes provided on the cell sheet transplantation device; and (4) attaching to the immobilized transplantation site the detached cultured cell sheet attached to the cell sheet capture surface, by weakening a force of adhesion between the cell adhesion surface and the cultured cells.
(l) The method as recited in (k), wherein at step (2), wherein the cell sheet transplantation device is put on another cultured cell sheet to pick up a stack of multiple cell sheets, and thereafter the cell sheet transplantation device is transferred to the transplantation site.
(m) The method as recited in any one of (k) and (1), wherein the transplantation site is present in a biological tissue.
(n) The method as recited in any one of (k) to (m), wherein the transplantation site is a site composed of a partially or totally damaged or deficient tissue.
(o) A treatment method wherein a sheet of cultured cells is transplanted onto an affected site in a biological tissue which is composed of a partially or totally damaged or deficient tissue, using the cell sheet transplantation device as recited in any one of (a) to (j).

### ADVANTAGEOUS EFFECTS OF INVENTION

By using the inventive cell sheet transplantation tool having a convex-shaped cell adhesion surface, cultured cells in a given range on a cell culture substrate can be detached effectively, and the detached cultured cells can be simply attached again. Thus, the cultured cells can be simply and accurately transferred to any site to which they are desired to be transferred.

### BRIEF DESCRIPTIONS OF DRAWINGS

FIG. 1 shows the cell sheet transplantation device having suction holes as mentioned in Example 1. In this figure, (a) is a view as seen from the top, and (b) is a view as seen from the bottom.
FIG. 2 shows the cell sheet transplantation device having suction holes as mentioned in Example 1. In this figure, (a) is a view as seen from the top, and (b) is a view as seen from the bottom.
FIG. 3 shows the cell sheet transplantation device having suction holes as mentioned in Example 1. In this figure, (a) is a view as seen from the top, and (b) is a view as seen from the bottom.
FIG. 4 shows the cell sheet transplantation device having suction holes as mentioned in Example 1. In this figure, (a) is a view as seen from the top, and (b) is a view as seen from the bottom.
FIG. 5 shows the specific dimensions of the device disclosed in Example 1 and FIG. 3.
FIG. 6 shows the result of prototyping the device designed in Example 1 and in FIG. 2.
FIG. 7 shows another mode of the device designed in Example 1 and in FIG. 2, which is provided with a handle.
FIG. 8 shows the device for immobilizing a cell sheet by tying it up with a string as mentioned in Example 1.
FIG. 9 shows the device that can transplant three cell sheets at a time as mentioned in Example 1.
FIG. 10 shows the device that can transplant six cell sheets at a time as mentioned in Example 1.
FIG. 11 shows the transplantation device made with a soft material as mentioned in Example 2.
FIG. 12 shows that the device disclosed in Example 2 and FIG. 11 is soft.
FIG. 13 shows that the device disclosed in Example 2 and FIG. 11 can closely adhere to the surface of a pig's heart.

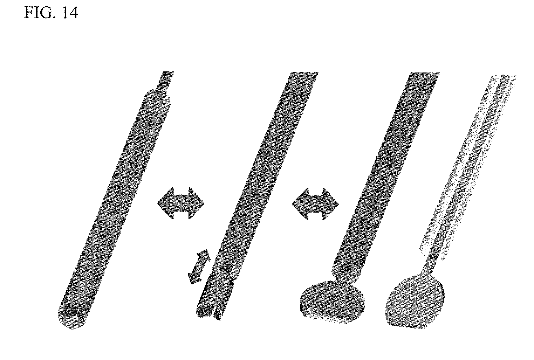

FIG. 14 shows the transplantation device that can be housed in a pipe as mentioned in Example 3.

### DESCRIPTION OF EMBODIMENTS

The present invention relates to a transplantation device for securely transplanting cells cultured in the form of sheet onto a transplantation site. The device is provided with suction holes for immobilizing the transplantation site. In this invention, when the suction holes are contacted with the transplantation site and suction is applied, a curved surface as of a biological tissue can be flattened, and the flattened surface is advantageous for subsequent transplantation steps of a cell sheet. The number of the suction holes in the device is not particularly limited, but it is preferred for the purpose of this invention that three or more suction holes should be provided in a flat. The shape of the suction holes, the spacing between them, and their positions with respect to the flat are also not particularly limited, and it is sufficient if the conditions are optionally adjusted depending on the intended transplantation site. Also, the strength of suction may be at any level without particular limitation as long as the transplantation site can be immobilized in a flat, and it is sufficient if the condition is optionally adjusted depending on the state of the transplantation site. In the process, strongly sucking the transplantation site is not preferred in the method of this invention, since the site will then be deteriorated. Further, the means for applying suction is not particularly limited, and a vacuum pump is commonly used. Furthermore, the material used to make the transplantation device is not particularly limited, and examples include, but are not particularly limited to, polyurethane, polyethylene elastomers, silicon resins, Teflon®, rubbers, polyethylene, polypropylene, polyethylene terephthalate, and metals. Among them, polyurethane, polyethylene elastomers, and silicon resins are advantageous in this invention because they are moderately-soft, elastic bodies that can be adapted to a tissue with a curved surface.

The transplantation tool of the present invention is provided with a cell sheet capture surface in a plane in the same direction as the above-mentioned suction holes. The material used to make the capture surface is not particularly limited, and examples include, but are not particularly limited to, polyurethane, polyethylene elastomers, silicon resins, Teflon®, rubbers, polyethylene, polypropylene, polyethylene terephthalate, and metals. Among them, polyurethane, polyethylene elastomers, and silicon resins are advantageous in this invention because they are moderately-soft, elastic bodies that can be adapted to a tissue with a curved surface. The material of the capture surface may or may not be the same as that of the above-mentioned suction holes. The capturing method is not particularly limited, and can be exemplified by slight sucking, immobilizing with a string or the like, and attachment using a cell adhesive material. For example, in the case of slight sucking, it is only sufficient if suction holes are provided on the cell sheet capture surface. In the process, the number of the suction holes in the tool is not particularly limited, but it is preferred for the purpose of this invention that three or more suction holes should be provided in a plane. The shape of the suction holes, the spacing between them, and their positions with respect to the plane are also not particularly limited, and it is sufficient if the conditions are optionally adjusted depending on the intended cell sheet. Also, the strength of suction may be at any level without particular limitation as long as the cell sheet can be immobilized in a plane, and it is sufficient if the condition is optionally adjusted depending on the state of the cell sheet. In the process, strongly sucking the cell sheet is not preferred in the method of this invention, since the cell sheet will then be deteriorated. Further, the means for applying suction is not particularly limited, and a vacuum pump is commonly used.

The transplantation device of the present invention comprises, in a flat in the same direction, (1) a planar surface portion for capturing a cell sheet while maintaining a sheet-shaped form, and (2) suction holes for immobilizing a transplantation site by suction, the suction holes being positioned around the planar surface portion. The flat may constitute a device with its shape retained or a device that can be housed in a pipe such that it can be put in or out of the pipe depending on the need. The latter case is advantageous in that the device will then be typically usable in endoscopes, whereby a cell sheet can be transplanted while invasion to a recipient's affected area is reduced.

In the present invention, one or two or more of, for example, a cell adhesive protein, a cell adhesive peptide, and a hydrophilic polymer may also be applied to the cell sheet capture surface. Among them, the cell adhesive protein can be one or two or more of fibrin gel, fibronectin, laminin, collagen, gelatin, and the like. The cell adhesive peptide can be one or two or more of RGD peptide, RGDS peptide, GRGD peptide, GRGDS peptide, and the like. The method for immobilizing the cell adhesive protein or cell adhesive peptide on the cell adhesion surface is not particularly limited; for example, it is sufficient to perform physical adsorption by a known conventional protocol such as applying an aqueous solution of the cell adhesive protein or cell adhesive peptide. The amount of the cell adhesive protein or cell adhesive peptide immobilized on the cell sheet capture surface may be any amount without particular limitation as long as said protein or peptide is immobilized in a sufficient amount to attach the cells desired to be transferred, and the immobilization amount is at least 0.005 µg/cm², preferably at least 0.01 µg/cm², and more preferably at least 0.02 µg/cm². The amount of the cell adhesive protein or cell adhesive peptide immobilized may be determined according to any conventional protocol; for example, it may be determined by directly measuring the cell adhesion surface using the FT-IR-ATR method, or by a method in which a cell adhesive protein or cell adhesive peptide that has been labeled beforehand is immobilized using the same procedure and estimation is made based on the amount of the labeled cell adhesive protein or cell adhesive peptide immobilized on the cell adhesion surface, and any other methods may also be used.

The hydrophilic polymer used in the present invention may be a homopolymer or a copolymer. Examples include, but are not particularly limited to, polyacrylamide, polydimethylacrylamide, polyacrylic acid and a salt thereof; hydrous gels such as polyhydroxyethyl methacrylate, polyhydroxyethyl acrylate, polyvinyl alcohol, polyvinyl pyrrolidone, cellulose, and carboxymethyl cellulose; and temperature-responsive gels whose hydration state changes with the temperature.

The hydrophilic polymer used in the present invention may also be a temperature-responsive polymer. The temperature-responsive polymer may be a homopolymer or a copolymer, and examples of the polymer include polymers described in Japanese Unexamined Patent Application Publication No. H02-211865. Specifically, such polymers are typically prepared by homopolymerization or copolymerization of the following monomers. Examples of monomers that can be used include (meth)acrylamide compounds, N-(or N,N-di)alkyl-substituted (meth)acrylamide derivatives, and vinyl ether derivatives. Copolymers can be made of any two or more of the above-mentioned monomers. Alternatively, they may be prepared by copolymerization of any of the above-mentioned monomers with any other monomer than said monomers, or by graft polymerization or copolymerization of polymers. A mixture of polymers or copolymers may also be used. Such polymers may also be crosslinked as long as their inherent properties are not impaired. The method for coating a surface of a substrate with various polymers is not particularly limited, and the coating can typically be performed according to the method disclosed in JP H02-211865. More specifically, the coating can be performed by subjecting the substrate and such a monomer or polymer as mentioned above to electron beam (EB) irradiation, γ-ray irradiation, ultraviolet ray irradiation, plasma treatment, corona treatment, or organic polymerization reaction, or by physical adsorption through spread coating, kneading or the like. The amount of the hydrophilic polymer immobilized on the cell adhesion surface may be any amount without particular limitation as long as said polymer is immobilized in a sufficient amount to attach the cells desired to be transferred, and the immobilization amount is at least 0.5 µg/cm², preferably at least 1.0 µg/cm², and more preferably at least 1.5 µg/cm². The amount of the hydrophilic polymer immobilized may be determined according to any conventional protocol; for example, it may be determined by directly measuring the cell adhesion surface using the FT-IR-ATR method, or by a method in which a hydrophilic polymer that has been labeled beforehand is immobilized using the same procedure and estimation is made based on the amount of the labeled hydrophilic polymer immobilized on the cell adhesion surface, and any other methods may also be used.

The cell adhesion surface to be provided in the cell sheet transplantation tool used in the present invention may be decided as appropriate depending on the size of the cultured cells or cultured cell sheet desired to be transferred, and this is not limited in any way. Also, the size of the cell sheet transplantation tool having the cell adhesion surface may be decided as appropriate depending on the size of the cell adhesion surface. Further, the shape of the cell sheet transplantation tool is not particularly limited, and the tool may be provided with a grip required to transfer the tool or with a mechanism that enables connection with some other apparatus.

If the cell sheet capture surface is convex shaped, the following is what occurs when the cell adhesion surface of the transplantation equipment is brought close to the cultured cells on the surface of a cell culture substrate: the most convex portion of the cell adhesion surface can come into first contact with the cultured cells; as the cell adhesion surface is brought closer, the area of contact between the cell adhesion surface and the cultured cells grows larger, with the most convex portion at the center, until it extends over the whole cell culture surface to be detached. By contacting the cell adhesion surface and the cultured cells in such a manner, they can be adhered with no air bubbles trapped between them, resulting in effective detachment of the cultured cells. In this process, the shape of the convex portion of the cell adhesion surface is not particularly limited, and it is sufficient if any portion of the cell adhesion surface is more convex than the other portions, but in order that a wide enough range of the cultured cells can be detached from the cell culture substrate, the most convex portion of the cell adhesion surface is preferably positioned at the center of the cell adhesion surface. As referred to herein, the term "center of the cell adhesion surface" means the central area of the cell adhesion surface if the most convex portion is forms a spot or a flat having the spot at the center, or this term means the center line area including the center of the cell adhesion surface if the most convex portion forms a line or a flat having the line at the center.

According to the present invention, the convex-shaped cell contact surface is brought into contact with the cultured cell surface, starting with the convex portion of the cell contact surface. In this process, all or part of the convex portion may be used. In either case, the convex portion of the cell contact surface comes into first contact with the cultured cell surface, and as the cell adhesion surface is brought closer to the cultured cell surface, the area of contact between the cell adhesion surface and the cultured cell surface becomes flat enough to establish contact. Speaking of the dimensions of the convex portion: within the scope of applications to cell adhesion, the height of the most convex portion is suitably in the range of 0.5-5 mm, preferably in the range of 0.8-3 mm, more preferably in the range of 1.0-2.5 mm, and most preferably in the range of 1.2-2.0 mm. A height below 0.5 mm is not desirable because this is the same as the case where the cell adhesion surface is flat and not all of the cells desired to be transferred can be necessarily detached. A height above 5 mm is also not desirable because a distortion that occurs when the convex-shaped cell adhesion surface eventually turns flat or, in some cases, the pressure being applied to flatten the convex-shaped cell adhesion surface will become an impact on the cultured cells.

In the present invention, the proportion of the convex area relative to the whole cell adhesion surface is not particularly limited but is suitably in the range of 40-100%, preferably in the range of 50-100%, more preferably in the range of 70-100%, and most preferably in the range of 80-100%. Extensive studies have shown that a proportion below 40% is not preferred in this invention because there will then be many cases where air bubbles may be trapped between the cell adhesion surface and the cultured cells.

Furthermore, the shape of the convex portion in the present invention is not particularly limited in any way where it is viewed from the side facing the cell adhesion surface or viewed vertically to the cell adhesion surface. Referring to the case of a shape as viewed vertically, for example, the whole cell adhesion surface to be utilized may be gradually convex, whether continuously or stepwise.

The cells to be used in the present invention can be any one type of cells among corneal epithelial cells, corneal endothelial cells, retinal pigment cells, epidermal keratinocytes, oral mucosal cells, conjunctival epithelial cells, cardiomyocytes, fibroblasts, vascular endothelial cells, hepatocytes, skeletal myoblasts, mesenchymal stem cells, pneumonocytes, mesothelial cells, chondrocytes, synoviocytes, osteocytes, periodontal ligament cells, and other stem cells, or a mixture of two or more types thereof; the type of the cells is not limited at al. The origin of the cells is not particularly limited, and examples include human, dog, cat, rabbit, rat, pig, and sheep, but if the cultured cells of this invention are used for treatment of humans, it is preferred to use human-derived cells.

The medium to be used for cell culture in the present invention is not particularly limited as long as it is the one commonly used for the cells to be cultured, but if the resulting cultured cells are used for treatment of humans, it is desirable to use a medium having components that are clear as to their origins or approved as pharmaceutical products.

The form of the culture substrate in the present invention is not particularly limited, and examples include forms like a dish, a multiplate, a flask, a cell insert, and a flat membrane.

The present invention provides a method for transferring cultured cells, in which cultured cells on the cell culture substrate are attached to the cell adhesion surface provided on the cell sheet transplantation device to detach the cultured cells from the cell culture substrate, and then a force of adhesion between the cell adhesion surface of the cell sheet transplantation device and the cultured cells is weakened to thereby reattach the detached cultured cells to a given site. It has been found that according to this method, detachment of the cultured cells from the cell culture substrate and reattachment of the detached cultured cells can be conducted in a simple manner and, thus, the cultured cells can be simply and accurately transferred to any site to which they are desired to be transferred.

In order to transfer the cultured cells in the present invention, it is necessary as a first step to attach the cultured cells present on the cell culture substrate to the cell adhesion surface provided on the cell sheet transplantation device. The method for effecting the attachment is not limited at all, but since the cell sheet transplantation device of this invention is provided with the cell adhesion surface, it is only sufficient to place this surface on the cultured cells desired to be transferred and allow it to stand. If a cell adhesive protein or cell adhesive peptide is applied to the cell adhesion surface of this invention, the cultured cells will be attached to the cell sheet transplantation device through the cell adhesive protein or cell adhesive peptide. If a hydrophilic polymer is applied to the cell adhesion surface, the cultured cells will be physically attached to the cell sheet transplantation device by the water-absorbing capacity of the hydrophilic polymer or the hydrophilicity/hydrophobicity of the polymer layer surface of the cell adhesion surface. In the process, various steps may be taken for the purpose of promoting attachment: for example, a load may be applied to the cultured cells to the extent to which no burden is put on them, or sufficient time may be taken until attachment is effected. Other steps for promoting attachment of the cultured cells may also be taken, such as increasing/decreasing the amount of a medium or changing a culture temperature. The attachment may also be performed automatically using a Z stage that enables an attachment operation in the vertical direction.

The cultured cells attached to the cell sheet transplantation device by the above-mentioned method can be freely transferred together with the cell sheet transplantation device to any desired site and transplanted onto the site. In the process, the transfer of the cultured cells is preferably conducted aseptically to prevent their contamination. The transfer may also be conducted under humidification to prevent drying of the attached cells.

The present invention also provides a technique by which the cultured cells transferred by the above-mentioned method are replaced on a desired site, where they are attached again. The method for performing the reattachment is not particularly limited. According to a conventional protocol, the transferred cultured cells are put on the desired site, and then an adhesion between the cell adhesion surface of the cell sheet transplantation device and the cultured cells is weakened to release the cell sheet transplantation device from the cultured cells, whereupon the reattachment process is completed. In the process, if it does not particularly matter that the cell adhesion surface of the cell sheet transplantation device remains unremoved, detachment of the cell adhesion surface and the cultured cells together may also be effected at the position of the cell adhesion surface of the cell sheet transplantation device. The method for weakening the adhesion between the cell adhesion surface of the cell sheet transplantation device and the cultured cells is exemplified by the following methods. If the cell adhesion surface is a cell adhesive protein or cell adhesive peptide, the adhesion may be weakened by, for example, adding an amino acid, peptide, protein, or the like that attach the cells more strongly than said adhesive protein or peptide, or introducing plenty of a medium. If the cell adhesion surface of the cell sheet transplantation device is a hydrophilic polymer, the cultured cells can be detached by introducing plenty of a medium to reduce the water-absorbing capacity of the hydrophilic polymer, or making the polymer layer surface of the cell adhesion surface sufficiently hydrophilic. Other various steps may also be taken for the purpose of promoting reattachment to a desired site; for example, a load may be applied to the cultured cells to the extent to which no burden is put on them, or sufficient time may be taken until attachment is effected, or a culture temperature may be changed.

As referred to herein, the transplantation site is not particularly limited; for example, it may be a surface of a culture substrate, a surface of an *in vivo* tissue, a surface of an *in vitro* tissue, a top of other cultured cells, or a top of another cultured cell sheet. As used herein, the terms "surface of an *in vivo* tissue" and "surface of an *in vitro* tissue" can be exemplified by the surfaces of those tissues derived from human, dog, cat, rabbit, rat, pig, sheep, and the like, and these terms are not limited depending on the origins of the tissues. The term "other cultured cells" refers to any one type of cells among corneal epithelial cells, epidermal keratinocytes, oral mucosal cells, conjunctival epithelial cells, cardiomyocytes, fibroblasts, vascular endothelial cells, and hepatocytes, or a mixture of two or more types thereof; the type of the cells is not limited at al. If the cultured cells of this invention are used for treatment of humans, it is preferred to use human-derived cells.

If the surface of the cell culture substrate is covered with a temperature-responsive polymer, cultured cells can be detached in the form of sheet from the surface of the cell culture substrate merely by changing a culture temperature as disclosed in WO 02/08387, so by using the technique of the present invention, the detachment, transfer, and reattachment operations can be conducted in a simple and accurate manner. In this case, the temperature-responsive polymer with which to coat the surface of the substrate has an upper or lower critical solution temperature of 0 to 80°C, more preferably 20 to 50°C, in an aqueous solution. An upper or lower critical solution temperature higher than 80°C is undesirable since it may cause death of cells. An upper or lower critical solution temperature lower than 0°C is also undesirable since it usually causes an extreme decrease in cell growth rate or death of cells.

The temperature-responsive polymer used in the present invention may be a homopolymer or a copolymer. Examples of these polymers include polymers described in JP H02-211865. Specifically, such polymers are typically prepared by homopolymerization or copolymerization of the following monomers. Examples of monomers that can be used include (meth)acrylamide compounds, N-(or N,N-di)alkyl-substituted (meth)acrylamide derivatives, and vinyl ether derivatives. Copolymers can be made of any two or more of the above-mentioned monomers. Alternatively, they may be prepared by copolymerization of any of the above-mentioned monomers with any other monomer than said monomers, or by graft polymerization or copolymerization of polymers. A mixture of polymers or copolymers may also be used. Such polymers may also be crosslinked as long as their inherent properties are not impaired.

The method for coating the surface of the substrate with the temperature-responsive polymer is not particularly limited; for example, the method disclosed in JP H02-211865 can be followed. More specifically, the coating can typically be performed by subjecting the substrate and said monomer or polymer to electron beam (EB) irradiation, γ-ray irradiation, ultraviolet ray irradiation, plasma treatment, corona treatment, or organic polymerization reaction, or by physical adsorption through spread coating, kneading or the like. The coating amount of the temperature-responsive polymer can be in the range of 0.4 to 4.5 µg/cm², preferably 0.7 to 3.5 µg/cm² and more preferably 0.9 to 3.0 µg/cm². A coating amount lower than 0.2 µg/cm² is undesirable since it makes it difficult for cells to be detached from the polymer even if a stimulus is applied, leading to a significant deterioration in work efficiency. Also, a coating amount higher than 4.5 µg/cm² makes it difficult for cells to attach to the area of interest, preventing them from attaching adequately.

According to the present invention, in order that after cultured cells are detached in the form of sheet, the cultured cell sheet can be stacked on another sheet or transplanted onto an *in vivo* or *in vitro* tissue by using the cell sheet transplantation device, it is necessary that cells be cultured on the cell culture substrate coated with the temperature-responsive polymer before detachment of a sheet of the cultured cells. In the process, the temperature of a medium is not particularly limited as long as it is below the upper critical solution temperature, if any, of the above-mentioned polymer applied to the surface of a culture substrate or it is above the lower critical solution temperature, if any, of said polymer. Needless to say, it is inappropriate to perform culture in a low temperature range where no cultured cells grow or in a high temperature range where cultured cells die. The culture conditions other then temperature may be decided pursuant to any conventional protocol and are not particularly limited. For example, the medium to be used may be a medium supplemented with a known serum such as fetal bovine serum (FCS) or a serum-free medium not supplemented with such a serum.

In the method of the present invention, a sheet of cultured cells can be detached and harvested from the cell culture substrate coated with the temperature-responsive polymer by attaching the cultured cell sheet to the cell sheet transplantation device and bringing the temperature of the culture substrate surface to a temperature either above the upper critical solution temperature or below the lower critical solution temperature. The detachment of the cultured cell sheet may be performed in a culture solution in which the cells have been cultured, or in any other isotonic solution; the solution to be used can be selected depending on the purpose.

The cultured cell sheet detached from the cell culture substrate coated with the temperature-responsive polymer and picked up using the cell sheet transplantation device according to the present invention is characterized in that the cell sheet is not damaged by a protease as typified by dispase or trypsin during culture, that the basement membrane-like protein formed between the cells and the substrate during culture is also not enzymatically broken down, and that the cell sheet maintains a cell-cell desmosome structure so that it has high strength with few structural defects. Further, by using the cell sheet transplantation device, the cultured cell sheet can be stacked on another sheet or transferred to a diseased tissue in an accurate manner. These characteristics allow the cultured cell sheet as transplanted to graft to a diseased tissue in a satisfactory and accurate manner, thereby enabling efficient treatment.

As referred to herein, the method for immobilizing the cultured cell sheet onto a biological tissue is not particularly limited. The cultured cell sheet and the biological tissue may be immobilized by suturing, or alternatively, since the inventive cultured cell sheet quickly grafts to a biological tissue, suturing may not be required to immobilize the cultured cell sheet attached to a recipient's diseased site.

By using the inventive cell sheet transplantation device having suction holes, cultured cells in a given range on the cell culture substrate can be detached effectively, and the detached cultured cells can be simply transplanted again. Thus, the cultured cells can be simply and accurately transferred to any site to which they are desired to be transferred.

### EXAMPLES

The present invention will be described below in more detail by way of Examples, but these Examples do not limit this invention at all.

### [Example 1]

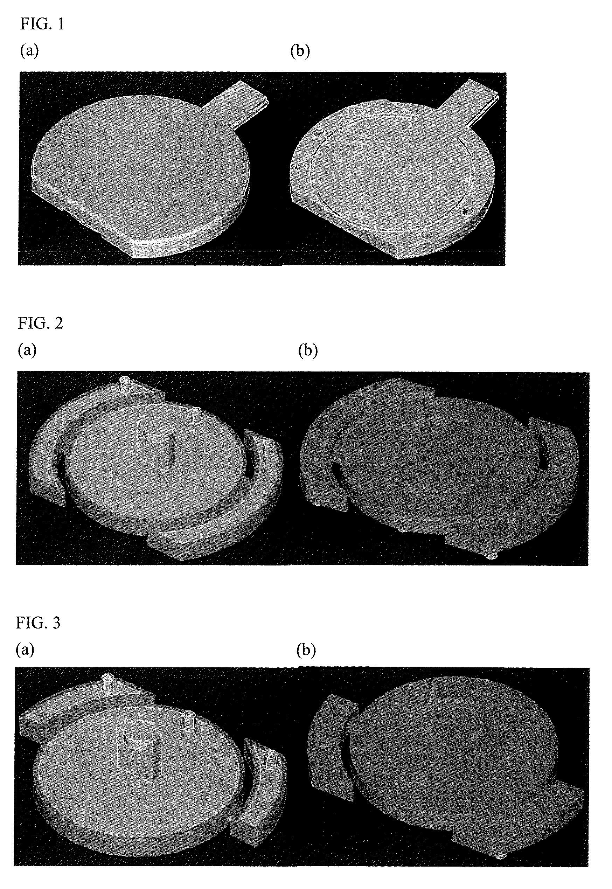

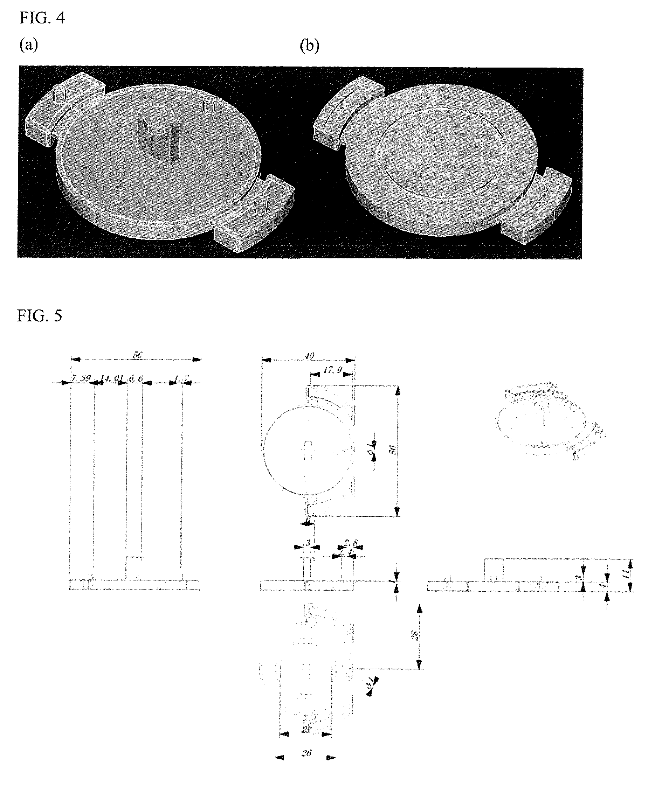

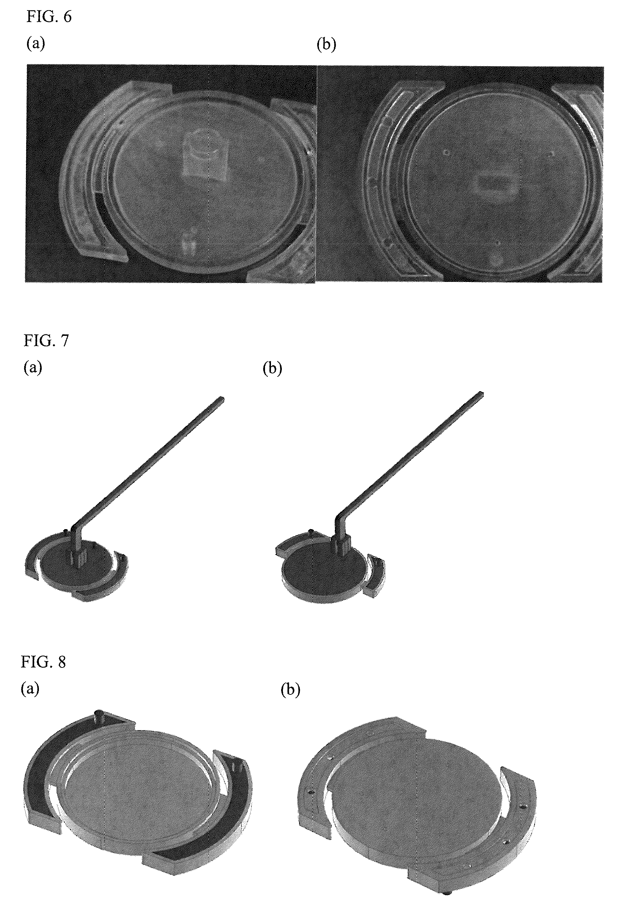

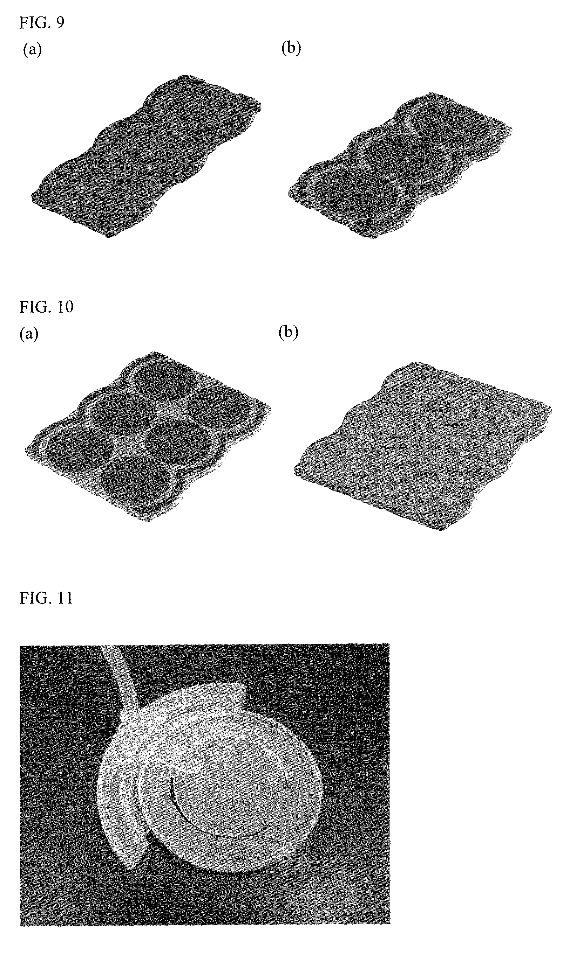

As specific examples of the cell sheet transplantation device of the present invention, four different shapes of devices having suction holes arranged in different ways were designed (FIGs. 1-4). In each of the figures, (a) shows an overview of a device, and (b) shows the bottom face which has suction holes and a cell sheet capture surface. FIG. 5 shows the specific dimensions of the device shown in FIG. 3. FIG. 6 shows the result of prototyping the device designed in FIG. 2. This prototype can be improved so as to make a cell sheet easier to transplant, by attaching a handle according to any of the two methods shown in FIG. 7. FIG. 8 shows the device that can immobilize a cell sheet by tying it up with a string. FIG. 9 shows a specific example of the device that can transplant three cell sheets at a time. FIG. 10 shows a specific example of the device that can transplant six cell sheets at a time.

### [Example 2]

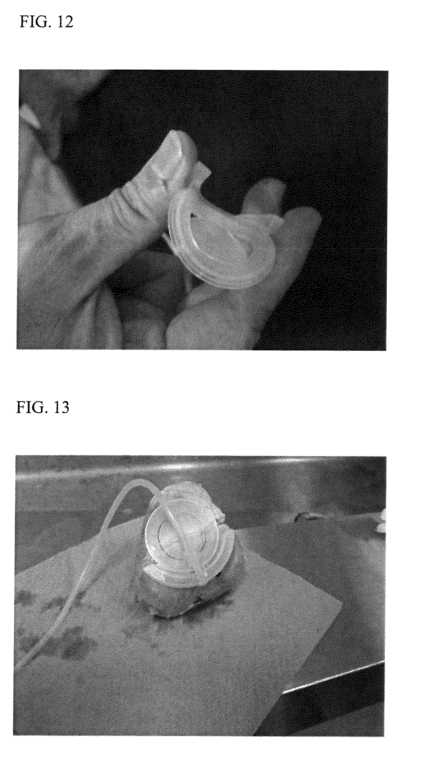

The cell sheet transplantation device of the present invention was molded with a soft silicon resin material. FIG. 11 shows an external top view of the whole of the produced transplantation device. The cell sheet capture area (the opposite face to that shown in this figure) has suction holes as referred to herein. FIG. 12 shows that the inventive product is made of a soft material. The result of experiment shows that the inventive product can closely adhere even to the spherical surface of a pig's heart (FIG. 13).

### [Example 3]

Another specific example of the transplantation device of the present invention was given. This transplantation device, which has a plane comprising: (1) a planar surface portion for capturing a cell sheet while maintaining a sheet-shaped form; and (2) suction holes for immobilizing a transplantation site by suction, the suction holes being positioned around the planar surface portion, can be housed in a pipe such that it can be put in and out of the pipe depending on the need.

### INDUSTRIAL APPLICABILITY

By using the cell sheet transplantation device of the present invention, cultured cells in a given range on a cell culture substrate can be detached effectively, and the detached cultured cells can be simply transplanted again. Thus, the cultured cells can be simply and accurately transferred to any site to which they are desired to be transferred. Further, by using a cell culture substrate with its surface coated with a temperature-responsive polymer, a cultured cell sheet showing extremely high engraftment with a biological tissue can be obtained. The cultured cell sheet obtained by the inventive method is strongly expected for clinical applications as in corneal transplantation, skin transplantation, treatment of corneal diseases, and treatment of ischemic heart diseases. Accordingly, the present invention is extremely useful in the fields of biology, medical science, etc., including cell engineering and medical engineering.

## Claims

1. A cell sheet transplantation device having a plane for transplanting a sheet of cultured cells, the jigdevice comprising, in the plane in the same direction, (1) a planar surface portion for capturing a cell sheet while maintaining a sheet-shaped form, and (2) suction holes for immobilizing a transplantation site by suction, the suction holes being positioned around the planar surface portion.

2. The cell sheet transplantation device according to Claim 1, wherein the cell sheet transplantation device is plate-shaped.

3. The cell sheet transplantation device according to any one of Claims 1 and 2, wherein the cell sheet transplantation device is an elastic body.

4. The cell sheet transplantation device according to any one of Claims 1-3, wherein the planar surface portion for capturing a cell sheet is a cell adhesion surface for detaching cells cultured on a surface of a cell culture substrate and then reattaching the detached cultured cells onto another site.

5. The cell sheet transplantation device according to any one of Claims 1-4, wherein the planar surface portion for capturing a cell sheet has suction holes for immobilizing a cell sheet by suction.

6. The cell sheet transplantation device according to any one of Claims 1-5, wherein the planar surface portion for capturing a cell sheet is a convex-shaped elastic body which is brought into contact with the surface of the cell culture substrate in such a way that it starts contacting at a tip of a convex portion of the cell adhesion surface until it finally establishes contact with the whole of a given surface of the cell culture substrate.

7. The cell sheet transplantation device according to any one of Claims 1-6, wherein the planar surface portion for capturing a cell sheet has attached thereto one or two or more of a cell adhesive protein, a cell adhesive peptide, and a hydrophilic polymer.

8. The cell sheet transplantation device according to Claim 7, wherein the cell adhesive protein is composed of one or two or more of fibrin gel, fibronectin, laminin, collagen, and gelatin.

9. The cell sheet transplantation device according to Claim 7, wherein the hydrophilic polymer is a hydrous gel.

10. The cell sheet transplantation device according to Claim 7, wherein the hydrophilic polymer is a temperature-responsive polymer.

11. A method for using a cell sheet transplantation device, the method comprising: (1) attaching cultured cells present on a cell culture substrate to a planar surface portion for capturing a cell sheet, which is provided on the cell sheet transplantation device according to any one of Claims 1-9, to thereby detach a sheet of the cultured cells from the cell culture substrate; (2) transferring the cell sheet transplantation device to a transplantation site; (3) immobilizing the transplantation site by suction through suction holes provided on the cell sheet transplantation device; and (4) attaching to the immobilized transplantation site the detached cultured cell sheet attached to the cell sheet capture surface, by weakening a force of adhesion between the cell adhesion surface and the cultured cells.

12. The method according to Claim 11, wherein at step (2), wherein the cell sheet transplantation device is put on another cultured cell sheet to pick up a stack of multiple cell sheets, and thereafter the cell sheet transplantation device is transferred to the transplantation site.

13. The method according to any one of Claims 11 and 12, wherein the transplantation site is present in a biological tissue.

14. The method according to any one of Claims 11-13, wherein the transplantation site is a site composed of a partially or totally damaged or deficient tissue.
